# EUROPEAN PATENT SPECIFICATION

(11) **EP 2 800 573 B1**
(45) Date of publication and mention of the grant of the patent: **07.04.2021**
(21) Application number: 13733780.4
(22) Date of filing: 07.01.2013
(51) Int. Cl.: A61K 31/728, A61K 31/79, A61K 9/00, A61P 27/04

(54) **OPHTHALMIC COMPOSITION**
OPHTHALMISCHE ZUSAMMENSETZUNG
COMPOSITION OPHTALMIQUE

(30) Priority: 08.01.2012 US 201261584258 P
(43) Date of publication of application: 12.11.2014
(73) Proprietor: Medicure Technologies Ltd., 30889 Caesarea (IL)
(72) Inventor: ZOLOTARIOV, Eyal, 75682 Rishon Lezion (IL); ZOLOTARIOV, Zvi, 69016 Tel Aviv (IL)
(74) Representative: Lecomte & Partners
(86) International application number: PCT/IL2013/050019
(87) International publication number: WO 2013/102907

(56) References cited:
- EP-A1- 2 057 983
- EP-A2- 2 070 518
- WO-A1-89/03205
- WO-A2-2004/035005
- CN-A- 1 389 213
- US-A- 4 120 949

## Description

### FIELD OF THE INVENTION

This invention relates in general to ophthalmic compositions. In particular, it relates to low-viscosity topically administrable ophthalmic compositions for treating dry eyes.

### BACKGROUND OF THE INVENTION

Dry Eye Syndrome (DES) is a condition characterized by the inability of the eye to maintain a layer of tears sufficient to lubricate it properly. The national Eye Institute of the USA/Industry Workshop defines DES as "a disorder of a tear film due to tear deficiency or excessive tear evaporation which causes damage to the interpalpebral ocular surface and is associated with symptoms of ocular discomfort."

Many palliative treatments for dry eye syndrome have been developed over the course of the last half-century (see, for example, M. J. Doughty and S. Glavin, "Efficacy of Different Dry Eye Treatments with Artificial Tears or Ocular Lubricants: a Systematic Review," Ophthal. Physiol, Opt. 2009, 29, 573 - 583). In recent years, such treatments have tended either to include carbomer gels or hyaluronic acid as active ingredients.

South Korean patent KR100775065 discloses an ophthalmic composition comprising, *inter alia*, a carbomer (polyacrylate) and at least one of hyaluronic acid and povidone.

U.S. Pat. 7758883 discloses a three-layer "artificial tears" composition comprising an aqueous solution of polyvinyl acetate (0.5% - 10% by weight), polyvinyl alcohol (PVA) (0.5% - 10% by weight), polyvinyl pyrrolidone (PVP) (0.5% - 10% by weight), and a phospholipid (0.003% - 0.02% by weight).

U.S. Pat. Appl. 20040253202 discloses a topical ophthalmic composition comprising an aqueous solution of three polymeric ingredients, wherein the polymers include hydroxypropyl methylcellulose and a combination of two polymers selected from the group of combinations consisting of guar gum and a carboxyvinyl polymer; guar gum and hydroxyethyl cellulose; guar gum and dextran; hydroxyethyl cellulose and a carboxyvinyl polymer; and dextran and a carboxyvinyl polymer, provided that if the composition comprises a carboxyvinyl polymer then the composition does not contain sodium chloride or boric acid. A synergistic effect of the combination is claimed.

Chinese Patent Application CN1389213A discloses the use of sodium hyaluronate and polyvinyl pyrrolidone together to produce synergistic action and prepare said invented eye-moistening solution composition which possesses the effects of moistening eye region, making artificial tears, raising exchange of tears and lubricating soft contact lens to raise wearing comfort

U.S. Pat. Appl. 20040253280 discloses a topical opthalmic composition comprising an aqueous solution including a viscosity enhancing amount of combination of two polymers selected from the group consisting of hydroxypropyl methylcellulose and guar gum; hydroxypropyl methylcellulose and a carboxyvinyl polymer; a carboxyvinyl polymer and guar gum; hydroxypropyl methylcellulose and hydroxyethylcellulose; hyaluronic acid and hydroxypropyl methylcellulose; and hyaluronic acid and guar gum, provided that if the composition comprises a carboxyvinyl polymer then the composition does not contain sodium chloride or boric acid. A synergistic effect on the solution's viscosity is claimed.

U.S. Pat. Appl. 20080050335 discloses an ophthalmic composition without any carbomer, comprising 0.05 - 0.4% (w/v) hyaluronic acid and 0.25 - 4% (w/v) polyvinyl alcohol. The composition is further defined as having a viscosity of 20 - 150 centistoke and a pH of 5.0 - 8.5.

Artificial tears are usually delivered to the eye as drops or ointments, which are provided in intermittent doses rather than continuously as are natural tears. To overcome this problem the artificial tears are composed of ingredients that increase contact time with the ocular surface. These ingredients are designed to have mucoadhesive properties and are generally formulated as viscous gels. One problem faced by palliative treatments for dry eye, particularly those that lack a carbomer gel, is the high viscosity of the ingredients. In many cases, if the composition contains a sufficiently high concentration of the active ingredients, it is so viscous that application is uncomfortable for the patient, the high viscosity of the composition leading to problems such as irritation, blurred vision, stickiness of the eyelids, or a sensation of heavy eyelids.

Various formulation strategies have been implemented in attempts to overcome the disadvantages of the use of highly viscous materials. One such strategy is the use of a less viscous formulation, that relies on its mucoadhesive properties to remain on the surface of the eye. Some examples of polymers that are mucoadhesive by physical or chemical interaction that have found use in compositions for ocular treatment include hyaluronic acid (HA), polyvinyl pyrrolidone (PVP), and polyvinyl alcohol (PVA).

Sodium hyaluronate, which is found in the aqueous and vitreous humor, mimics mucin and supports the epithelial cells of the cornea. This material is finding increasing use in artificial tear preparations primarily due to three useful properties. First, it is a viscoelastic polymer that exhibits non-Newtonian fluid properties, mimicking the natural tear film in becoming more elastic during blinking, thus increasing spreading and improving aqueous lubrication of the anterior ocular surface epithelial tissues. Second, it has anti-inflammatory properties, which can be useful in treatment of the surface inflammation prevalent in DES. Third, it has mucoadhesive properties as well.

PVP is beneficial in mucin deficiency. Its high dipole moment and polarity cause it to become hydrated in aqueous solution, helping maintenance of the water layer, and it has the ability to interact with a variety of surfaces by hydrogen or electrostatic bonding. These properties are useful for palliation of DES, but PVP alone does not provide an effective treatment.

PVA, which has been shown to be beneficial in the treatment of lipid, aqueous and mucin layer deficiency is widely used in artificial tear solutions. PVA reduces surface tension and is used as a wetting agent, offering improved aqueous lubrication to the epithelial tissues. In addition, PVA has a long retention time.

While these polymers are mucoadhesive, their adhesion is frequently insufficient for effective use in treatments for DES. In addition, attempts to use HA alone have run into the problem that this ingredient tends to be irritating to the eye when used in concentrations sufficiently high to treat DES. Thus, there remains a long- felt need for a palliative treatment for DES that is both low-viscosity and non-irritating, and that has improved mucoadhesive properties relative to compositions known in the art for the treatment of DES.

### SUMMARY OF THE INVENTION

The invention disclosed herein is designed to meet this long-felt need. In particular, a topically administrable ophthalmic composition is disclosed that comprises hyaluronic acid (HA) or a pharmaceutically acceptable salt thereof and polyvinyl pyrrolidone (PVP) as the active ingredients. The observed synergistic effect between these two ingredients enables formulation of a composition in which they are present in low concentrations, namely on the order of 0.1 % HA (normally as sodium hyaluronate) and 5% PVP. In some embodiments of the invention, it further comprises a borate or citrate buffer, which is present in much lower concentrations than in comparable topical ophthalmic compositions known in the art, and an inorganic salt to control the osmolarity.

Thus, the invention relates to an ophthalmic composition consisting of: an aqueous solution of hyaluronic acid (HA) or a pharmaceutically acceptable salt thereof; polyvinyl pyrrolidone (PVP); a quantity of an inorganic salt sufficient to bring the composition to a predetermined osmolarity; strong acid or strong base as necessary to bring the composition to a predetermined pH; and optionally, a buffer; characterized in that said ophthalmic composition is topically administrable; and said ophthalmic composition is characterized by a concentration of HA or a pharmaceutically acceptable salt thereof of about 0.1 % w/v and a concentration of PVP of about 5% w/v, wherein the term "about" refers to quantities within ±25% of the stated quantity.

It is a further object of this invention to disclose an ophthalmic composition as defined above, wherein said PVP has an average molecular weight of between 50,000 and 65,000 daltons.

It is a further object of this invention to disclose an ophthalmic composition as defined in any of the above, wherein said HA or pharmaceutically acceptable salt thereof and PVP are present in amounts that provide a synergistic reduction in viscosity.

It is a further object of this invention to disclose an ophthalmic composition as defined in any of the above, wherein said HA or pharmaceutically acceptable salt thereof and PVP are present in amounts that provide a synergistic increase in therapeutic effectiveness.

It is a further object of this invention to disclose an ophthalmic composition as defined in any of the above, wherein said composition does not comprise a therapeutically effective amount of any substance that is therapeutically effective against dry eye other than HA, pharmaceutically acceptable salts of HA, or PVP.

It is a further object of this invention to disclose an ophthalmic composition as defined in any of the above, additionally comprising a buffer.

It is a further object of this invention to disclose such an ophthalmic composition, wherein said buffer is chosen from the group consisting of (a) a borate - boric acid buffer; and (b) a citrate - citric acid buffer.

It is a further object of this invention to disclose such an ophthalmic composition, wherein said buffer is a borate - boric acid buffer, and further wherein said buffer comprises boric acid and sodium tetraborate decahydrate in substantially equal concentrations (w/v).

It is a further object of this invention to disclose such an ophthalmic composition, wherein said buffer is a citrate - citric acid buffer comprising about 18.8 g trisodium citrate per liter of composition and about 84 mg citric acid per liter of composition.

It is a further object of this invention to disclose an ophthalmic composition as defined in any of the above, wherein sufficient acid or base has been added to adjust the pH to a predetermined value.

It is a further object of this invention to disclose an ophthalmic composition as defined in any of the above, further comprising an inorganic salt; in cases where the ophthalmic composition comprises a buffer, the inorganic salt is a salt that is not the salt of a weak acid.

It is a further object of this invention to disclose an ophthalmic composition as defined in any of the above, wherein the osmolarity of said ophthalmic composition is about 0.3 osmol L⁻¹.

It is a further object of this invention to disclose such an ophthalmic composition as defined in any of the above, wherein sufficient NaCl is added to adjust the osmolarity to about 0.3 osmol L⁻¹.

It is a further object of this invention to disclose such an ophthalmic composition, wherein said composition comprises about 0.1% w/v sodium hyaluronate, about 5% w/v PVP, about 0.19% w/v boric acid, and about 0.19% w/v sodium tetraborate decahydrate.

It is a further object of this invention to disclose such an ophthalmic composition, wherein said composition comprises about 0.1% w/v sodium hyaluronate, about 5% w/v PVP, about 1.88% w/v trisodium citrate, and about 0.008% w/v citric acid.

It is a further object of this invention to disclose an ophthalmic composition as defined in any of the above, wherein the pH of said ophthalmic composition is between about 5 and about 8.

It is a further object of this invention to disclose an ophthalmic composition as defined in any of the above, wherein the pH of said ophthalmic composition is about 7.4.

It is a further object of this invention to disclose an ophthalmic composition as defined above for use in the treatment of dry eye syndrome.

The invention further relates to a method of producing an ophthalmic composition, said method consisting of: preparing an aqueous solution comprising HA or a pharmaceutically acceptable salt thereof and PVP; adding a quantity of an inorganic salt that is not the salt of a weak acid sufficient to adjust the osmolarity to a predetermined level; mixing until said inorganic salt that is not the salt of a weak acid is dissolved; optionally, adding at least one substance selected from the group consisting of inorganic salts and buffers; optionally adding sufficient acid or base to adjust the pH to a predetermined pH; and adding additional water as necessary to yield a composition characterized by HA and PVP concentrations of about 0.1% w/v and about 5% w/v, respectively, wherein the term "about" refers to quantities within ±25% of the stated quantity.

It is a further object of this invention to disclose such a method, wherein said step of preparing an aqueous solution comprising HA or a pharmaceutically acceptable salt thereof and PVP comprises preparing an aqueous solution of HA or pharmaceutically acceptable salt thereof; preparing an aqueous solution of PVP; and mixing the two solutions.

It is a further object of this invention to disclose a method as defined in any of the above, additionally comprising preparing an aqueous solution of PVA; and wherein said step of preparing an aqueous solution comprising HA or a pharmaceutically acceptable salt thereof and PVP comprises adding HA or a pharmaceutically acceptable salt thereof and PVP to said aqueous solution of PVA.

It is a further object of this invention to disclose a method as defined in any of the above, additionally comprising adding a buffer.

It is a further object of this invention to disclose such a method, wherein said step of adding a buffer comprises a step of adding a buffer chosen from the group consisting of (a) a borate - boric acid buffer and (b) a citrate - citric acid buffer.

It is a further object of this invention to disclose such a method, wherein said step of adding a buffer comprises a step of adding substantially equal weights of boric acid and sodium tetraborate decahydrate.

It is a further object of this invention to disclose such a method, wherein said step of adding a buffer comprises adding trisodium citrate and citric acid in a weight ratio of about 18800 : 84.

It is a further object of this invention to disclose such a method, wherein said inorganic salt is NaCl.

It is a further object of this invention to disclose such a method, wherein said predetermined level is about 0.3 osmol L⁻¹.

It is a further object of this invention to disclose a method as defined in any of the above, additionally comprising a step of adding sufficient acid or base to adjust the pH to a predetermined level.

It is a further object of this invention to such a method, wherein said step adding sufficient acid or base to adjust the pH to a predetermined level comprises a step of adding sufficient acid or base to adjust the pH to between about 5 and about 8.

It is a further object of this invention to such a method, wherein said step adding sufficient acid or base to adjust the pH to a predetermined level comprises a step of adding sufficient acid or base to adjust the pH to 7.4.

It is a further object of this invention to disclose a method of treating dry eye syndrome, comprising administering to a patient in need the ophthalmic composition as defined in any of the above.

It is a further object of this invention to disclose such a method, additionally comprising a step of providing said ophthalmic composition in the form of drops.

### DETAILED DESCRIPTION OF THE PREFERRED EMBODIMENTS

In the following description, various aspects of the invention will be described. For the purposes of explanation, specific details are set forth in order to provide a thorough understanding of the invention. The invention is defined by the claims. Any subject-matter falling outside the scope of the claims is provided for information purposes only, even if it is referred to in the following as being part of the invention or relating to an embodiment of the invention.

Unless otherwise stated, all concentrations are stated as percent w/v.

As used herein, the term "about" refers to quantities within ±25% of the stated quantity.

The invention herein disclosed provides a topically administrable ophthalmic composition and method of production thereof that can provide effective treatment for dry eye syndrome. The composition comprises, in some preferred embodiments, hyaluronic acid (HA) or a pharmaceutically acceptable salt thereof and polyvinyl pyrrolidone (PVP) as the sole components that are pharmaceutically effective against dry eye syndrome; in other preferred embodiments, the composition comprises HA or a pharmaceutically acceptable salt thereof, PVP, and polyvinyl alcohol (PVA) as the sole components that are pharmaceutically effective against dry eye syndrome. Non-limiting examples of inactive ingredients in the composition include buffers and inorganic salts.

The inventors have discovered that, surprisingly, a composition containing both HA and PVP has better mucoadhesive properties than a composition containing an equivalent amount of either component alone, and furthermore, such a composition has superior mucoadhesive properties to those that would be expected from a mere combination of the properties of the individual components. Thus, in one embodiment of the invention, the ophthalmic composition is an aqueous solution of PVP with HA or a pharmaceutically acceptable salt thereof. Without wishing to be bound by theory, one plausible explanation for the synergistic effect of the combination of HA and PVP may be that the ability of PVP to form complexes with large anionic polymers such as sodium hyaluronate provides improved mucoadhesive properties relative to those of the individual components.

In typical embodiments of the invention, the pharmaceutically acceptable salt of HA used is sodium hyaluronate. In preferred embodiments of the invention, the average molecular weight of the PVP is between 50,000 and 65,000 Daltons, in the most preferred embodiments, about 58,000 Daltons; PVP with properties appropriate for use in the invention herein disclosed is commercially available, e.g. as Plasdone-C30 (International Specialty Products).

The inventors have further discovered, surprisingly, a that a composition containing PVA and PVP shows improved efficacy against DES than would be expected from a composition containing an equivalent amount of either component alone, or that would be expected from a combination of the properties of the two components. Thus, in some preferred embodiments of the invention, the ophthalmic composition comprises an aqueous solution of PVA, PVP, and HA or a pharmaceutically acceptable salt thereof.

Without wishing to be bound by theory, a plausible explanation for the synergistic effect of the combination of PVP and PVA may be that the two polymers attract each other by hydrogen and electrostatic bonds, forming a PVA-PVP complex that can serve as a polar phase in the lipid layer of the tears. This polar phase can interact with the both lipid layer and the aqueous layer, stabilizing the outer lipid layer and inhibiting evaporation of tear film. This complex may also act in the mucin layer, where the hydration properties of PVP are retained, while the retention time of the composition is enhanced by the PVA.

In some more preferred embodiments of the invention, the solution also contains a buffer. In yet more preferred embodiments of the invention, the buffer used is either a borate - boric acid buffer or a citrate - citric acid buffer. In the most preferred embodiments of the invention, the total amount of material added (weak acid + salt thereof) in order to form the buffer is <0.5% w/v (borate - boric acid buffer) or <2% w/v (citrate - citric acid buffer) relative to the total amount of the composition. Embodiments of the invention that include PVA typically incorporate a citrate - citric acid buffer.

In preferred embodiments of the invention, an inorganic salt is added to adjust the osmolarity. In the most preferred embodiments of the invention, the osmolarity of the composition is adjusted to about 0.3 osmol L⁻¹ by addition of the salt. In preferred embodiments of the invention, the inorganic salt is not the salt of a weak acid; in the most preferred embodiments, sodium chloride is used.

In preferred embodiments of the invention, the pH of the composition is between about 5 and about 8. In the most preferred embodiments of the invention, the pH is about 7.4. In some embodiments, the pH is adjusted to a predetermined value (between 5 and 8 in preferred embodiments, about 7.4 in the most preferred embodiments) by addition of a small quantity, typically on the order of 0.1% (v/v) relative to the total volume of composition, of a solution of strong acid (typically 0.1 - 0.5 M aqueous HCl) or strong base (typically 0.1 - 0.5 M aqueous NaOH) to the solution containing HA or a pharmaceutically acceptable salt thereof and PVP (and inorganic salt and/or buffer in those embodiments that contain either or both of these components).

In some preferred embodiments of the invention, the ophthalmic composition contains about 0.05% - 0.2% w/v sodium hyaluronate, 0.6% - 5% w/v PVP, about 0.19% w/v boric acid, and about 0.19% w/v sodium tetraborate decahydrate.

In other preferred embodiments of the invention, the ophthalmic composition contains about 0.05% - 0.2% sodium hyaluronate, about 0.6% - 5% w/v PVP, and about 1.4% w/v PVA. In other preferred embodiments of the invention, the ophthalmic composition contains about 0.05% - 0.2% w/v sodium hyaluronate, 0.6% - 5% w/v PVP, about 1.4% w/v PVA, about 1.88% w/v trisodium citrate, and about 0.0084% w/v citric acid.

It is also within the scope of the invention to disclose a method for producing the ophthalmic composition. According to one embodiment of this method, an aqueous solution of HA or a pharmaceutically acceptable salt thereof and PVP is prepared, either by preparing a solution of one of the components and adding the second component to the solution or by preparing two separate solutions and mixing them. In preferred embodiments, components of a buffer (typically substantially equal weights of boric acid and sodium tetraborate decahydrate; in some embodiments, citric acid and trisodium citrate) are then added. If necessary, sufficient water is then added to dilute the concentrations of the active ingredients to predetermined levels, typically about 0.05% - 0.2% w/v HA or pharmaceutically acceptable salt thereof and 0.5% - 6% w/v PVP. In preferred embodiments, the osmolarity is then adjusted (typically to about 0.3 osmol L⁻¹) by addition of salt (in preferred embodiments, NaCl). In preferred embodiments, the pH is then adjusted by addition of acid or base (typically 0.1 - 0.5 M HCl or 0.1 - 0.5 NaoH) if necessary. In preferred embodiments, the pH is adjusted to a value between about 5 and about 8. In the most preferred embodiments, the pH is adjusted to a value of about 7.4.

According to a second embodiment of the method, an aqueous solution of PVA in water is prepared. In preferred embodiments, PVA is added to water at a temperature above room temperature, most preferably at about 60 °C. In preferred embodiments, the temperature is maintained at above room temperature until the PVA is completely dissolved. In more preferred embodiments, the temperature is maintained between about 60 °C and about 80 °C until the PVA is completely dissolved. In the most preferred embodiments, the temperature is maintained at about 60 °C until the PVA is completely dissolved. In embodiments in which the solution is prepared at elevated temperature, it is then allowed to cool (preferably with stirring), typically to about 25 °C. In preferred embodiments, components of a buffer (typically citric acid and trisodium citrate) are then added. PVP and HA or a pharmaceutically acceptable salt thereof are then added to the solution. If necessary, sufficient water is then added to dilute the concentrations of the active ingredients to predetermined levels, typically about 1.4% PVA, about 0.05% - 0.2% w/v HA or pharmaceutically acceptable salt thereof, and 0.5% - 6% w/v PVP. In preferred embodiments, the osmolarity is then adjusted (typically to about 0.3 osmol L⁻¹) by addition of salt (in preferred embodiments, NaCl). In preferred embodiments, the pH is then adjusted by addition of acid or base (typically 0.1 - 0.5 M HCl or 0.1 - 0.5 NaoH) if necessary. In preferred embodiments, the pH is adjusted to a value between about 5 and about 8. In the most preferred embodiments, the pH is adjusted to a value of about 7.4.

To some extent, the method does not depend on the exact order in which the components of the composition are added and mixed. As a non-limiting example, the buffer can be added after all of the active ingredients have been mixed rather than after the preparation of a solution containing only one of them. In general, final adjustments of the osmolarity and pH will be the last steps of the preparation, but intermediate additions of salt, acid, and base, taking place between other steps of the invention, are contemplated by the inventors as being within the scope of the invention.

It is also within the scope of the invention to disclose a method for treating dry eye syndrome. The method comprises administering to a patient in need the ophthalmic composition of the present invention, preferably in the form of drops, until the symptoms of dry eye syndrome are alleviated.

The following non-limiting examples are given to illustrate the invention herein disclosed and to provide a description of the best modes contemplated by the inventors for its use. In addition to descriptions of several non-limiting embodiments of the invention, the examples also disclose several non-limiting embodiments of methods for preparation of the ophthalmic composition herein disclosed.

### EXAMPLE 1

A solution of 50 g PVP (Plasdone C-30) in 500 ml purified water was prepared, and visually inspected to confirm complete dissolution of the PVP. The solution was then diluted with an additional 400 ml of purified water. 1.0 g sodium hyaluronate was added, and visually inspected to confirm complete dissolution of the sodium hyaluronate. The solution was then stirred for approximately 10 minutes. 1.9 g boric acid was then added and the resulting solution stirred for 10 minutes. 1.9 g sodium tetraborate decahydrate was then added and the resulting solution stirred for an additional 10 minutes. Sufficient water was added to bring the total volume to 1000 ml. The osmolarity of the solution was then adjusted to ∼0.3 osmol L⁻¹ by addition of 7 g of NaCl, and 0.5 ml of concentrated HCl added to lower the pH to approximately 7.4. The final composition, comprising ∼0.1% sodium hyaluronate, ∼5% PVP, ∼0.19% H₃BO₃, ∼0.19% Na₂B₄O₇·10H₂O, and ∼0.7% NaCl, was then transferred to 100 ml bottles.

### Example 2 (reference example)

A solution of 1.0 g sodium hyaluronate in 400 ml purified water was prepared, and visually inspected to confirm complete dissolution of the sodium hyaluronate. A second solution of 6.0 g PVP (Plasdone C-30) in 400 ml was prepared, and visually inspected to confirm complete dissolution of the PVP. The two solutions were then combined and stirred for approximately 10 minutes. 1.9 g boric acid was then added and the resulting solution stirred for 10 minutes. 1.9 g of sodium tetraborate decahydrate was then added and the resulting solution stirred for 10 minutes. Sufficient water was added to bring the total volume to 1000 ml. The osmolarity of the solution was then adjusted to ∼0.3 osmol L⁻¹ by addition of 7 g of NaCl, and the pH adjusted to ∼7.4 by addition of concentrated HCl. The final product, an aqueous solution comprising ∼0.1% sodium hyaluronate, ∼0.6% PVP, ∼0.19% H₃BO₃, ∼0.19% Na₂B₄O₇·10H₂O, and ∼0.7% NaCl, was then transferred to 100 ml bottles.

### EXAMPLE 3

50.0 g of PVP (Plasdone C-30) was added in several stages to 900 ml of purified water with stirring. Complete dissolution of the PVP was confirmed by visual inspection. 1.0 g of sodium hyaluronate was then added with stirring. The resulting solution was inspected visually to confirm complete dissolution of the sodium hyaluronate. 84 mg of citric acid were added and the resulting solution stirred for 10 minutes. 18.8 g of trisodium citrate were added, and the resulting solution stirred for an additional 10 minutes. Sufficient purified water was added to bring the volume to 1000 ml. The osmolarity was adjusted to ∼0.3 osmol L⁻¹ by addition of 3 g of NaCl. The pH was adjusted to 7.4 by addition of ∼6 ml of an 0.5 M NaOH solution. The resulting composition (∼0.1% sodium hyaluronate, ∼5% PVP, ∼0.008% citric acid, ∼1.8% trisodium citrate, and ∼0.3% NaCl) was then transferred to 10 ml bottles.

### Example 4 (reference example)

6.0 g of PVP (Plasdone C-30) was added in stages to 900 ml of purified water with stirring. Complete dissolution of the PVP was confirmed by visual inspection. 1.0 g of sodium hyaluronate was then added with stirring, and the resulting solution inspected visually to confirm complete dissolution of the sodium hyaluronate. 84 mg of citric acid were added and the resulting solution stirred for 10 minutes. 18.8 g of trisodium citrate were added, and the resulting solution stirred for an additional 10 minutes. Sufficient water was then added to bring the total volume to 1000 ml. The osmolarity of the solution was adjusted to ∼0.3 osmol L⁻¹ by addition of 4.1 g of NaCl. The pH was then adjusted to 7.4 by addition of ∼1.4 g of an 0.5 M NaOH solution. The resulting composition (∼0.1% sodium hyaluronate, ∼0.6% PVP, ∼0.008% citric acid, ∼1.8% trisodium citrate, and ∼0.4% NaCl) was then transferred to 10 ml bottles.

### Example 5 (reference example)

14.0 g of PVA was added in several stages to 700 ml of purified water at 60 °C and stirred for 1 hour using a mechanical stirrer, during which time the temperature was maintained at a constant 60 °C. The resulting solution was visually inspected to confirm complete dissolution of the PVA, and then cooled to 25 °C with continuous stirring. 50.0 g of PVP (Plasdone C-30) was added to the solution and stirred until the PVP was completely dissolved, as confirmed by visual inspection. 1.0 g of sodium hyaluronate was then added to the solution and stirred until the sodium hyaluronate completely dissolved, as confirmed by visual inspection. Sufficient water was then added to bring the total volume to 1000 ml and the resulting solution stirred for 10 minutes. The osmolarity was adjusted to ∼0.3 osmol L⁻¹ by addition of approximately 7.8 g of NaCl. The solution was then stirred for an additional 10 minutes. The pH was then adjusted to 7.4 by addition of several drops of an aqueous solution of NaOH (0.5 M), resulting in an aqueous solution of ∼0.1% sodium hyaluronate, ∼5.0% PVP, ∼1.4% PVA, and ∼0.8% NaCl. The resulting composition was filtered through a 0.2 µm filter prior to storage in sealed containers.

### Example 6 (reference example)

14.0 g of PVA was added in several stages to 700 ml of purified water at 60 °C and stirred for 1 hour using a mechanical stirrer, during which time the temperature was maintained at a constant 60 °C. The resulting solution was visually inspected to confirm complete dissolution of the PVA, and then cooled to 25 °C with continuous stirring. 6.0 g of PVP (Plasdone C-30) was added to the solution and stirred until the PVP was completely dissolved, as confirmed by visual inspection. 1.0 g of sodium hyaluronate was then added to the solution and stirred until the sodium hyaluronate completely dissolved, as confirmed by visual inspection. Sufficient water was added to bring the total volume to 1000 ml. The osmolarity was adjusted to ∼0.3 osmol L⁻¹ by addition of 9.3 g of NaCl followed by stirring for 10 minutes. The pH was then adjusted to 7.4, by addition of 1.5 ml of an aqueous NaOH solution (0.1 M) and 1 ml of 0.1 M HCl, resulting in an aqueous solution of ∼0.1% sodium hyaluronate, ∼0.6% PVP, ∼1.4% PVA, and ∼0.8% NaCl. The resulting composition was filtered through a 0.2 µm filter prior to storage in sealed containers.

### Example 7 (reference example)

14.0 g of PVA was added in several stages to 700 ml of purified water at 60 °C and stirred for 1 hour using a mechanical stirrer, during which time the temperature was maintained at a constant 60 °C. The solution was visually inspected to confirm complete dissolution of the PVA and then cooled to 25 °C with continuous stirring. 84 mg of citric acid was then added, and the resulting solution stirred for an additional 10 minutes. 18.8 g of trisodium citrate was then added, and the resulting solution stirred for an additional 10 minutes. 50.0 g of PVP (Plasdone C-30) was added to the solution and stirred until the PVP was completely dissolved, as confirmed by visual inspection. 1.0 g of sodium hyaluronate was then added to the solution and stirred until the sodium hyaluronate completely dissolved, as determined by visual inspection. Sufficient water was then added to bring the total volume to 1000 ml. The osmolarity was adjusted to ∼0.3 osmol L⁻¹ by addition of 2 g NaCl. The pH was then adjusted to 7.4 by addition of ∼4.5 ml of an aqueous NaOH solution (0.5 M), resulting in an aqueous solution of ∼0.1% sodium hyaluronate, ∼5.0% PVP, ∼1.4% PVA, ∼0.188% sodium citrate, ∼0.008% citric acid, and ∼0.5% NaCl. The resulting composition was filtered through a 0.2 µm filter prior to storage in sealed containers.

### Example 8 (reference example)

14.0 g of PVA was added in several stages to 700 ml of purified water at 60 °C and stirred for 1 hour using a mechanical stirrer, during which time the temperature was maintained at a constant 60 °C. The solution was visually inspected to confirm complete dissolution of the PVA and then cooled to 25 °C with continuous stirring. 84 mg of citric acid was then added, and the resulting solution stirred for an additional 10 minutes. 18.8 g of trisodium citrate was then added, and the resulting solution stirred for an additional 10 minutes. 6.0 g of PVP (Plasdone C-30) was added to the solution and stirred until the PVP was completely dissolved, as confirmed by visual inspection. 1.0 g of sodium hyaluronate was then added to the solution and stirred until the sodium hyaluronate completely dissolved. Sufficient water was then added to bring the total volume to 1000 ml and the resulting solution stirred for a further 10 minutes. The osmolarity was adjusted to ∼0.3 osmol L⁻¹ by addition of 2.5 g NaCl. The pH was then adjusted to 7.4 by addition of 7.9 g of an aqueous NaOH solution (0.1 M), resulting in an aqueous solution of ∼0.1% sodium hyaluronate, ∼0.6% PVP, ∼1.4% PVA, ∼0.188% sodium citrate, ∼0.008% citric acid, and ∼0.8% NaCl. The resulting composition was filtered through a 0.2 µm filter prior to storage in sealed containers.

### Example 9 (Compositions comprising PVA are not part of the invention)

Tests were made of the long-term stability of the compositions of the present invention. The concentrations of the active ingredients in the compositions were assayed, and some critical physical properties measured, at the time of preparation and then again after 3 months' incubation at 40 °C. Table 1 summarizes results for compositions comprising HA and PVP, and Table 2 summarizes results for compositions comprising HA, PVP, and PVA.

**TABLE 1**

| | HA, PVP, citrate buffer | | HA, PVP, citrate buffer | |
|---|---|---|---|---|
| appearance | initial clear liquid | incubation at 40 °C for 3 months clear liquid | initial clear liquid | incubation at 40 °C for 3 months clear liquid |
| HA (assay) | 0.10% | 0.099% | 0.10% | 0.097% |
| PVP (assay) | 0.59% | 0.58% | 5.14% | 5.24% |
| pH | 7.4 | 7.4 | 7.3 | 7.2 |
| sp. gr. (g/mL) | 1.012 | 1.016 | 1.024 | 1.024 |
| osmolarity (mOsm/L) | 310.3 | 321.3 | 310.3 | 322.0 |
| viscosity (mPa·s) | 6.8 | - | 9.9 | - |

**TABLE 2**

| | HA, PVP, PVA, citrate buffer | | HA, PVP, PVA, citrate buffer | |
|---|---|---|---|---|
| | initial | incubation at 40 °C for 3 months | initial | incubation at 40 °C for 3 months |
| appearance | clear liquid | clear liquid | clear liquid | clear liquid |
| HA (assay) | 0.11% | 0.11% | 0.10% | 0.10% |
| PVP (assay) | 0.77% | 0.77% | 5.24% | 5.43% |
| PVA (assay) | 1.43% | 1.40% | 1.65% | 1.60% |
| pH | 7.0 | 6.5 | 7.2 | 6.5 |
| sp. gr. (g/mL) | 1.017 | 1.018 | 1.027 | 1.024 |
| osmolarity (mOsm/L) | 284.6 | 305.3 | 317.3 | 332.0 |
| viscosity (mPa·s) | 6.8 | - | 9.9 | - |

Viscosity measurements were only made at the time of the preparation of the compositions.

As can be seen from the results summarized in the tables, the compositions of the present invention show excellent long-term stability.

## Claims

1. An ophthalmic composition consisting of:
an aqueous solution of hyaluronic acid (HA) or a pharmaceutically acceptable salt thereof;
polyvinyl pyrrolidone (PVP);
a quantity of an inorganic salt sufficient to bring the composition to a predetermined osmolarity;
strong acid or strong base as necessary to bring the composition to a predetermined pH; and
optionally, a buffer;
**characterized in that**
said ophthalmic composition is topically administrable ; and
said ophthalmic composition is **characterized by** a concentration of HA or a pharmaceutically acceptable salt thereof of about 0.1% w/v and a concentration of PVP of about 5% w/v, wherein the term "about" refers to quantities within ±25% of the stated quantity.

2. The ophthalmic composition according to claim 1, wherein at least one of the following is true:
said PVP has an average molecular weight of between 50,000 and 65,000 daltons;
said predetermined pH is between 5 and 8; and
said inorganic salt is not a salt of a weak acid.

3. The ophthalmic composition according to claim 1, wherein said inorganic salt is NaCl.

4. The ophthalmic composition according to claim 1, wherein the osmolality of said ophthalmic composition is about 0.3 osmol L⁻¹.

5. The ophthalmic composition according to claim 1, wherein said buffer is selected from the group consisting of (a) a borate - boric acid buffer; and (b) a citrate - citric acid buffer.

6. The ophthalmic composition according to claim 5, wherein said buffer is a borate - boric acid buffer comprising boric acid and sodium tetraborate decahydrate in substantially equal concentrations (w/v).

7. The ophthalmic composition according to claim 1, wherein said composition contains about 0.1% w/v sodium hyaluronate, about 5% w/v PVP, about 0.19% w/v boric acid, and about 0.19% w/v sodium tetraborate decahydrate; and sufficient NaCl to bring the osmolarity to about 0.3 osmol L-1.

8. A method of producing an ophthalmic composition, said method consisting of:
preparing an aqueous solution comprising HA or a pharmaceutically acceptable salt thereof and PVP;
adding a quantity of an inorganic salt that is not the salt of a weak acid sufficient to adjust the osmolarity to a predetermined level;
mixing until said inorganic salt that is not the salt of a weak acid is dissolved;
optionally, adding at least one substance selected from the group consisting of inorganic salts and buffers;
optionally adding sufficient acid or base to adjust the pH to a predetermined pH;
and
adding additional water as necessary to yield a composition **characterized by** HA and PVP concentrations of about 0.1% w/v and about 5% w/v, respectively, wherein the term "about" refers to quantities within ±25% of the stated quantity.

9. The method according to claim 8, wherein said step of preparing an aqueous solution comprising HA or a pharmaceutically acceptable salt thereof and PVP comprises:
preparing an aqueous solution of HA or pharmaceutically acceptable salt thereof;
preparing an aqueous solution of PVP; and,
mixing the two solutions.

10. The method according to claim 8, wherein said step of adding sufficient acid or base to adjust the pH to a predetermined pH comprises adding sufficient acid or base to adjust the pH to between 5 and 8.

11. The method according to claim 10, wherein said step of adding sufficient acid or base to adjust the pH to a predetermined pH comprises adding sufficient acid or base to adjust the pH to 7.4.

12. The method according to claim 8, wherein said step of adding at least one substance selected from the group consisting of inorganic salt and buffers comprises a step of adding a buffer chosen from the group consisting of (a) a borate - boric acid buffer and (b) a citrate - citric acid buffer.

13. The method according to claim 12, wherein said step of adding a buffer comprises a step selected from the group consisting of:
adding substantially equal weights of boric acid and sodium tetraborate decahydrate; and,
adding trisodium citrate and citric acid in a weight ratio of about 18800 : 84.

14. The method according to claim 8, wherein at least one of the following is true:
said inorganic salt is NaCl; and,
said predetermined level is about 0.3 osmol L⁻¹.

15. The ophthalmic composition according to any one of claims 1-7 for use in the treatment of dry eye syndrome.

## Patentansprüche

1. Ophtalmische Zusammensetzung bestehend aus:
einer wässrigen Lösung aus Hyaluronsäure (HA) oder einem pharmazeutisch verträglichen Salz davon;
Polyvinylpyrrolidon (PVP);
einer Menge eines anorganischen Salzes, die ausreicht, um die Zusammensetzung auf eine vorbestimmte Osmolarität zu bringen;
einer starken Säure oder starken Base nach Bedarf, um die Zusammensetzung auf einen vorbestimmten pH-Wert zu bringen und
optional einen Puffer;
**dadurch gekennzeichnet, dass**
die ophthalmische Zusammensetzung topisch verabreichbar ist, und
die ophthalmische Zusammensetzung **gekennzeichnet ist durch** eine Konzentration von HA oder einem pharmazeutisch verträglichen Salz davon von etwa 0,1 % (Gew./Vol.) und eine Konzentration von PVP von etwa 5 % (Gew./Vol.), wobei sich der Begriff "etwa" auf Mengen innerhalb von ±25 % der angegebenen Menge bezieht.

2. Ophthalmische Zusammensetzung nach Anspruch 1, wobei mindestens eine der folgenden Bedingungen erfüllt ist:
das PVP hat ein durchschnittliches Molekulargewicht zwischen 50.000 und 65.000 Dalton;
der vorbestimmte pH-Wert liegt zwischen 5 und 8; und
das anorganische Salz ist kein Salz einer schwachen Säure.

3. Ophthalmische Zusammensetzung nach Anspruch 1, wobei das anorganische Salz NaCl ist.

4. Ophthalmische Zusammensetzung nach Anspruch 1, wobei die Osmolalität der ophthalmischen Zusammensetzung etwa 0,3 osmol L⁻¹ beträgt.

5. Ophthalmische Zusammensetzung nach Anspruch 1, wobei der Puffer aus der Gruppe, bestehend aus (a) einem Borat-Borsäure-Puffer und (b) einem Citrat-Zitronensäure-Puffer, ausgewählt wird.

6. Ophthalmische Zusammensetzung nach Anspruch 5, wobei der Puffer ein Borat-Borsäure-Puffer ist, der im Wesentlichen gleiche Konzentrationen (Gew./Vol.) von Borsäure und Natriumtetraborat-Decahydrat umfasst.

7. Ophthalmische Zusammensetzung nach Anspruch 1, wobei die Zusammensetzung etwa 0,1 % (Gew./Vol.) Natriumhyaluronat, etwa 5 % (Gew./Vol.) PVP, etwa 0,19 % (Gew./Vol.) Borsäure und etwa 0,19 % (Gew./Vol.) Natriumtetraborat-Decahydrat enthält und ausreichend NaCl, um die Osmolarität auf etwa 0,3 Osmol L⁻¹ zu bringen.

8. Verfahren zur Herstellung einer ophthalmischen Zusammensetzung, wobei das Verfahren darin besteht:
Herstellung einer wässrigen Lösung, die HA oder ein pharmazeutisch verträgliches Salz davon und PVP enthält;
Hinzufügen einer Menge eines anorganischen Salzes, das kein Salz einer schwachen Säure ist, und ausreicht
die Osmolarität auf einen vorbestimmten Wert einzustellen;
Vermischen, bis das anorganische Salz, das kein Salz einer schwachen Säure ist, aufgelöst ist;
optional Hinzufügen mindestens einer Substanz, ausgewählt aus der Gruppe der anorganischen Salze und Puffer;
optional Hinzufügen von ausreichend Säure oder Base, um den pH-Wert auf einen vorbestimmten pH-Wert einzustellen; und
Hinzufügen von zusätzlichem Wasser nach Bedarf, um eine Zusammensetzung zu erhalten, die durch HA und PVP-Konzentrationen von etwa 0,1 % (Gew./Vol.) bzw. etwa 5 % (Gew./Vol.) gekennzeichnet ist, wobei sich der Begriff "etwa" auf Mengen innerhalb von ±25 % der angegebenen Menge bezieht.

9. Verfahren nach Anspruch 8, wobei der Herstellungsschritt einer wässrigen Lösung, die HA oder ein pharmazeutisch verträgliches Salz davon und PVP enthält, Folgendes umfasst:
Herstellung einer wässrigen Lösung aus HA oder einem pharmazeutisch verträglichem Salz davon;
Herstellung einer wässrigen Lösung aus PVP und
Vermischen der beiden Lösungen.

10. Verfahren nach Anspruch 8, wobei der Schritt des Hinzufügens von ausreichend Säure oder Base, um den pH-Wert auf einen vorbestimmten pH-Wert einzustellen, das Hinzufügen von ausreichend Säure oder Base umfasst, um den pH-Wert auf einen Wert zwischen 5 und 8 einzustellen.

11. Verfahren nach Anspruch 10, wobei der Schritt der Zugabe von ausreichend Säure oder Base, um den pH-Wert auf einen vorbestimmten pH-Wert einzustellen, das Hinzuf+gen von ausreichend Säure oder Base umfasst, um den pH-Wert auf 7,4 einzustellen.

12. Verfahren nach Anspruch 8, wobei der Schritt des Hinzufügens mindestens eine Substanz, ausgewählt aus der aus anorganischen Salze und Puffer bestehenden Gruppe, einen Schritt des Hinzufügens eines Puffers umfasst, der aus der Gruppe bestehend aus (a) einem Borat-Borsäure-Puffer und (b) einem Citrat-Zitronensäure-Puffer ausgewählt wurde.

13. Verfahren nach Anspruch 12, wobei der Schritt des Hinzufügens eines Puffers einen Schritt umfasst, ausgewählt aus der Gruppe, bestehend aus:
Hinzufügen von im Wesentlichen gleichen Gewichtsmengen an Borsäure und
Natriumtetraborat-Decahydrat und
Zugabe von Trinatriumcitrat und Zitronensäure in einem Gewichtsverhältnis von etwa 18800 : 84.

14. Verfahren nach Anspruch 8, wobei mindestens eine der folgenden Bedingungen erfüllt ist:
das anorganische Salz ist NaCl und
der vorbestimmte Wert ist etwa 0,3 osmol L⁻¹.

15. Ophthalmische Zusammensetzung nach einem der Ansprüche 1-7 zur Verwendung bei der Behandlung des trockenen Auge-Syndroms.

## Revendications

1. Composition ophtalmique, constituée de :
une solution aqueuse d'acide hyaluronique (HA) ou d'un sel acceptable sur le plan pharmaceutique de celui-ci ;
polyvinylpyrrolidone (PVP) ;
une quantité suffisante d'un sel inorganique pour porter la composition à une osmolarité prédéterminée ;
un acide fort ou une base forte en quantité nécessaire pour porter la composition à un pH prédéterminé ; et
optionnellement un tampon ;
**caractérisée en ce que**
ladite composition ophtalmique est administrable par voie topique ; et
ladite composition ophtalmique étant **caractérisée par** une concentration de HA ou d'un sel acceptable sur le plan pharmaceutique de celui-ci d'environ 0,1% p/v et une concentration en PVP d'environ 5% p/v, dans laquelle le terme « environ » se réfère à des quantités endéans ± 25% de la quantité indiquée.

2. Composition ophtalmique selon la revendication 1, dans laquelle au moins l'une des locutions qui suivent est vérifiée :
ladite PVP a un poids moléculaire moyen compris entre 50 000 et 65 000 daltons ;
ledit pH prédéterminé est compris entre environ 5 et 8 ; et
ledit sel inorganique n'est pas un sel d'un acide faible.

3. Composition ophtalmique selon la revendication 1, dans laquelle ledit sel inorganique est NaCl.

4. Composition ophtalmique selon la revendication 1, dans laquelle l'osmolalité de ladite composition ophtalmique est d'environ 0,3 osmol L⁻¹.

5. Composition ophtalmique selon la revendication 1, dans laquelle ledit tampon est choisi dans le groupe constitué par (a) un tampon borate - acide borique ; et (b) un tampon citrate - acide citrique.

6. Composition ophtalmique selon la revendication 5, dans laquelle ledit tampon est un tampon borate - acide borique comprenant l'acide borique et le tétraborate de sodium décahydrate à des concentrations sensiblement égales (p/v).

7. Composition ophtalmique selon la revendication 1, dans laquelle ladite composition comprend environ 0,1% p/v de hyaluronate de sodium, environ 5% p/v de PVP, environ 0,19% p/v d'acide borique, et environ 0,19% p/v de tétraborate de sodium décahydrate, et suffisamment de NaCl pour porter l'osmolarité à environ 0,3 osmol L⁻¹.

8. Procédé de production d'une composition ophtalmique, ledit procédé comprenant les étapes suivantes consistant à :
préparer une solution aqueuse comprenant le HA ou un sel acceptable sur le plan pharmaceutique de celui-ci et la PVP ;
ajouter une quantité suffisante d'un sel inorganique qui n'est pas un sel d'un acide faible pour ajuster l'osmolarité à un niveau prédéterminé ;
mélanger jusqu'à ce que le sel inorganique, qui n'est pas le sel d'un acide faible, est dissout ;
optionnellement ajouter au moins une substance choisie dans le groupe constitué par les sels inorganiques et les tampons ;
optionnellement ajouter une quantité suffisante d'un acide ou d'une base pour ajuster le pH à un pH prédéterminé ; et
ajouter de l'eau additionnelle juste nécessaire afin de fournir une composition **caractérisée par** des concentrations en HA et PVP d'environ 0,1 p/v et d'environ 5% p/v respectivement, dans laquelle le terme « environ » se réfère à des quantités endéans ± 25% de la quantité indiquée.

9. Procédé selon la revendication 8, dans lequel ladite étape de préparation d'une solution aqueuse comprenant le HA ou un sel acceptable sur le plan pharmaceutique de celui-ci et la PVP comprend :
préparer une solution aqueuse de HA ou d'un sel acceptable sur le plan pharmaceutique de celui-ci ;
préparer une solution aqueuse de PVP ; et
mélanger les deux solutions.

10. Procédé selon la revendication 8, dans lequel l'étape d'ajout d'une quantité suffisante d'acide ou de base pour ajuster le pH à un pH prédéterminé comprend l'ajout d'une quantité suffisante d'acide ou de base pour ajuster le pH entre environ 5 et 8.

11. Procédé selon la revendication 10, dans lequel l'étape d'ajout d'une quantité suffisante d'acide ou de base pour ajuster le pH à un pH prédéterminé comprend l'ajout d'une quantité suffisante d'acide ou de base pour ajuster le pH à 7,4.

12. Procédé selon la revendication 8, dans lequel ladite étape d'ajout d'au moins une substance choisie dans le groupe constitué par les sels inorganiques et les tampons comprend une étape d'ajout d'un tampon choisi dans le groupe constitué par (a) un tampon borate - acide borique et (b) un tampon citrate - acide citrique.

13. Procédé selon la revendication 12, dans lequel ladite étape d'ajout d'un tampon comprend une étape choisie dans le groupe constitué par :
un ajout de poids sensiblement égaux d'acide borique et de tétraborate de sodium décahydrate ; et
un ajout de citrate trisodique et d'acide citrique en un rapport pondéral d'environ 18800 : 84.

14. Procédé selon la revendication 8, dans lequel au moins l'une des locutions qui suivent est vérifiée :
ledit sel inorganique est le NaCl ; et
ledit niveau prédéterminé est d'environ 0,3 osmol L⁻¹.

15. Composition ophtalmique selon l'une quelconque des revendications 1 à 7 destinée à être utilisée dans le traitement du syndrome de l'œil sec.
